(19) Europäisches Patentamt
European Patent Office
Office européen des brevets

(11) EP 2 590 562 B1

(12) # EUROPÄISCHE PATENTSCHRIFT

(45) Veröffentlichungstag und Bekanntmachung des Hinweises auf die Patenterteilung:
**18.10.2017 Patentblatt 2017/42**

(21) Anmeldenummer: **11746442.0**

(22) Anmeldetag: **08.07.2011**

(51) Int Cl.:
***A61B 5/1455*** *(2006.01)*

(86) Internationale Anmeldenummer:
**PCT/EP2011/003401**

(87) Internationale Veröffentlichungsnummer:
**WO 2012/003989 (12.01.2012 Gazette 2012/02)**

(54) **BESTIMMEN DER KONZENTRATION EINES BLUTBESTANDTEILS IN EINER SCHLAUCHLEITUNG**

DETERMINING THE CONCENTRATION OF A BLOOD CONSTITUENT IN TUBING

DÉTERMINATION DE LA CONCENTRATION D'UN CONSTITUANT DU SANG DANS UNE CONDUITE FLEXIBLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.07.2010 DE 102010026723**

(43) Veröffentlichungstag der Anmeldung:
**15.05.2013 Patentblatt 2013/20**

(73) Patentinhaber: **Fresenius Medical Care Deutschland GmbH**
**61352 Bad Homburg v.d.H. (DE)**

(72) Erfinder: **ZHANG, Wei**
**97464 Niederwerrn (DE)**

(74) Vertreter: **Oppermann, Frank**
**OANDO Oppermann & Oppermann LLP**
**Washingtonstrasse 75**
**65189 Wiesbaden (DE)**

(56) Entgegenhaltungen:
**DE-A1-102009 005 402**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 590 562 B1

## Beschreibung

Die Erfindung betrifft ein Verfahren und eine Vorrichtung zum Bestimmen der Konzentration von Bestandteilen von Blut, insbesondere Hämoglobin, Hämatokrit, Glukose, Creatinin und Bilirubin, in einer Schlauchleitung, insbesondere einer Schlauchleitung eines extrakorporalen Blutkreislaufs einer extrakorporalen Blutbehandlungsvorrichtung. Darüber hinaus betrifft die Erfindung eine extrakorporale Blutbehandlungsvorrichtung mit einer Vorrichtung zum Bestimmen der Konzentration eines Blutbestandteils.

Zur Bestimmung der Konzentration von bestimmten Bestandteilen von Blut eines Patienten sind verschiedene Verfahren bekannt. Im Stand der Technik sind Verfahren zur Messung der Konzentration von Blutbestandteilen bekannt, die eine Entnahme einer Blutprobe voraussetzen. Es sind aber auch Messverfahren bekannt, bei denen die Konzentration von Blutbestandteilen in Blut gemessen werden kann, das durch eine Schlauchleitung strömt. Diese Verfahren finden insbesondere dann Anwendung, wenn bei einer extrakorporalen Blutbehandlung das Blut durch die Schlauchleitung eines extrakorporalen Blutkreislaufs fließt.

Bei einer extrakorporalen Blutbehandlung, insbesondere bei einer Blutbehandlung mit einer Hämodialysemaschine, ist die Überwachung der Konzentration von Hämoglobin im Blut des Patienten von Bedeutung. Häufige Messungen der Hämoglobinkonzentration ermöglichen durch eine Trendanalyse die frühzeitige Erkennung der Therapiewirkung und erlauben die frühzeitige Anpassung der Therapiemaßnahmen.

Die WO 2008/000433 A1 beschreibt ein Verfahren und eine Vorrichtung zur Bestimmung der Konzentration von bestimmten Blutbestandteilen in einer mit Blut gefüllten, im wesentlichen transparenten Schlauchleitung, insbesondere einer Schlauchleitung eines extrakorporalen Blutkreislaufs einer extrakorporalen Blutbehandlungsvorrichtung. Das bekannte Verfahren und die bekannte Vorrichtung erlauben insbesondere die Bestimmung der Hämoglobinkonzentration und des Hämatokrits. Während der Messung ist die Schlauchleitung zwischen zwei parallelen ebenen Anlageflächen eingespannt, so dass sich der Schlauch an den einander gegenüberliegenden Seiten verformt. Die Messung des Hämatokrit beruht auf der Transmission oder Streuung von elektromagnetischer Strahlung einer bestimmten Wellenlänge im Blut. Mit einem Emitter wird die elektromagnetische Strahlung durch die transparente Schlauchleitung in das Blut eingekoppelt, während mit einem Detektor die gestreute transmittierte elektromagnetische Strahlung aus dem Blut ausgekoppelt wird. Der Hämatokrit wird dann aus der Analyse der ausgekoppelten Strahlung bestimmt. Mit der bekannten Streulicht-, oder Transmissions-, oder Reflektionsmessung können aber auch die Konzentrationen von anderen Blutbestandteilen, beispielsweise Creatinin, Bilirubin, Glucose etc. bestimmt werden.

Die Intensität des ausgekoppelten Lichts ist nicht nur von der Konzentration der Blutbestandteile abhängig, sondern auch von anderen Faktoren. Daher kann die Konzentration der Blutbestandteile nicht ohne weiteres aus der Analyse des ausgekoppelten Lichts berechnet werden.

Bei der Messung spielen die Eigenschaften der Schlauchleitung eine Rolle, durch die das Licht ein- und ausgekoppelt wird. Die Schlauchleitung beeinflusst die optische Messung, da der Schlauch das Licht teilweise absorbiert und streut. Die Absorption des Lichts in der Schlauchleitung ist von den geometrischen Abmessungen der Schlauchleitung sowie der Beschaffenheit des Materials sowie der Oberflächenbeschaffenheit abhängig. Auf die Lichtabsorption hat auch das verwendete Sterilisationsverfahren einen gewissen Einfluss.

Aus der US 6 718 190 B2 ist eine Vorrichtung zur spektrofotometrischen Analyse von Eigenschaften des Bluts bekannt, die über einen Lichtemitter und einen Lichtdetektor verfügt. Da sich die Schlauchleitungen in Durchmesser und Wandstärke unterscheiden können, wird eine Kalibrierung für unterschiedliche Schlauchleitungen angestrebt. Es werden zwei unterschiedliche Verfahren zur Kalibrierung vorgeschlagen. Das erste Kalibrierverfahren beruht auf einer Änderung der Ultrafiltrationsrate, während das zweite Verfahren auf der Verwendung einer Indikatorlösung beruht. Soweit die Kalibrierung mit einer Indikatorlösung erfolgen soll, wird die Verwendung einer isotonischen Kochsalzlösung vorgeschlagen.

Die DE 693 32 325 T2 beschreibt ebenfalls eine Vorrichtung zur spektrofotometrischen Blutanalyse. Die Vorrichtung verfügt über mehrere in Längsrichtung der Schlauchleitung angeordnete Fotodioden, um das Problem zu lösen, dass Änderungen in der Natriumkonzentration zu signifikanten Änderungen der Lichttransmission führen.

Die DE 10 2009 005 402 A1 beschreibt ein Verfahren zur Bestimmung eines Blutbestandteils, insbesondere Hämoglobin, auf der Grundlage einer Transmissions- oder Streulichtmessung, wobei der Einfluss der Wandstärke und/oder der Materialeigenschaften der Schlauchleitung auf die Messung Berücksichtigung findet. Die Konzentration des Blutbestandteils wird in Abhängigkeit von einem Korrekturfaktor ermittelt, der mit einer separaten Transmissionsmessung bei einer anderen Flüssigkeit als Blut ermittelt wird.

Der Erfindung liegt die Aufgabe zugrunde, ein Verfahren bzw. eine Vorrichtung anzugeben, mit dem bzw. mit der nicht nur der Einfluss der Schlauchleitung auf die Bestimmung des Bestandteils von Blut in der Schlauchleitung auch dann verringert wird, wenn unterschiedliche Schlauchleitungen zum Einsatz kommen, sondern das bzw. die auch die Erkennung des zum Fördern von Blut verwendeten Schlauchleitungstyps erlaubt. Eine Aufgabe der Erfindung ist auch, eine extrakorporale Blutbehandlungsvorrichtung, insbesondere Hämodialysemaschine, mit einer derartigen Vorrichtung zu schaffen.

Die Lösung dieser Aufgaben erfolgt erfindungsgemäß mit den Merkmalen der unabhängigen Patentansprüche. Vorteilhafte Ausführungsformen der Erfindung sind Gegenstand der Unteransprüche.

Das erfindungsgemäße Verfahren und die erfindungsgemäße Vorrichtung beruhen auf der Korrektur des Einflusses der Schlauchleitung auf die Bestimmung der Konzentration des Blutbestandteils. Zur Bestimmung des Korrekturfaktors wird eine Referenzmessung mit einer anderen Flüssigkeit als Blut vorgenommen, um den Zusammenhang zwischen der Intensität der ausgekoppelten elektromagnetischen Strahlung bei einer Schlauchleitung, die mit Blut gefüllt ist, das den Blutbestandteil in einer bestimmten Konzentration enthält, und derselben Schlauchleitung, die mit einer anderen Flüssigkeit als Blut gefüllt ist, bestimmen zu können. Der Vorteil ist, dass nur eine einmalige Referenzmessung erforderlich ist, die werkseitig vorgenommen werden kann. Die erfindungsgemäße Vorrichtung verfügt über Mittel, mit denen der Zusammenhang zwischen der Intensität der ausgekoppelten elektromagnetischen Strahlung bei einer mit Blut gefüllten und der mit einer anderen Flüssigkeit als Blut gefüllten Schlauchleitung festgelegt ist. Bei diesen Mitteln kann es sich um einen Festwertspeicher handeln. Dieser Zusammenhang kann auch von einem Datenträger eingelesen werden.

Zum Bestimmen der Konzentration des Blutbestandteils in dem Blut, mit dem die Schlauchleitung gefüllt werden soll, wird die Schlauchleitung vor der Befüllung mit Blut mit einer anderen Flüssigkeit als Blut gefüllt, um die Intensität der ausgekoppelten elektromagnetischen Strahlung für die andere Flüssigkeit als Blut messen zu können. Die Bestimmung des Korrekturfaktors erfolgt dann auf der Grundlage des zuvor ermittelten Zusammenhangs zwischen der Intensität der ausgekoppelten elektromagnetischen Strahlung bei einer mit Blut gefüllten und der mit einer anderen Flüssigkeit als Blut gefüllten Schlauchleitung einerseits und auf der Grundlage der zuvor gemessenen Intensität der elektromagnetischen Strahlung, die aus der mit einer anderen Flüssigkeit als Blut gefüllten Schlauchleitung ausgekoppelt wird. Mit dem auf diese Weise ermittelten Korrekturfaktor können sämtliche Einflüsse der Schlauchleitung, insbesondere deren geometrische Abmessungen und Materialeigenschaften, auf die Bestimmung der Konzentration des Blutbestandteils minimiert oder kompensiert werden.

Wenn die Konzentrationen eines Bestandteils von Blut im Rahmen einer extrakorporalen Blutbehandlung bestimmt werden soll, kann die Messung mit einer anderen Flüssigkeit als Blut in der Schlauchleitung vor der Blutbehandlung vorgenommen werden. Als eine andere Flüssigkeit als Blut bietet sich beispielsweise eine Spülflüssigkeit oder Dialysierflüssigkeit an, da die Schlauchleitungen einer Blutbehandlungsvorrichtung vor der Durchführung der Blutbehandlung ohnehin mit einer Spülflüssigkeit, insbesondere isotonischen Kochsalzlösung, oder mit einer Dialysierflüssigkeit gespült werden. Grundsätzlich können aber auch andere Flüssigkeiten als eine Spülflüssigkeit oder Dialysierflüssigkeit verwendet werden.

Der Zusammenhang zwischen der Intensität der ausgekoppelten elektromagnetischen Strahlung bei einer mit Blut und der mit einer anderen Flüssigkeit als Blut gefüllten Schlauchleitung wird vorzugsweise durch eine mathematische Funktion beschrieben, die in einem Speicher abgelegt werden kann. Anstelle einer mathematischen Funktion ist es aber auch möglich, eine Vielzahl von Wertepaaren festzulegen, die in einem Speicher abgelegt werden. Allein entscheidend ist, dass der Zusammenhang bekannt ist, um bei der weiteren Auswertung Berücksichtigung finden zu können.

Zur Bestimmung des Zusammenhangs zwischen der Intensität der ausgekoppelten elektromagnetischen Strahlung bei einer mit Blut gefüllten und der mit einer anderen Flüssigkeit als Blut gefüllten Schlauchleitung werden vorzugsweise eine Mehrzahl von unterschiedlichen Schlauchleitungen, die sich in den geometrischen Abmessungen und/oder den Materialeigenschaften unterscheiden, einerseits mit Blut und andererseits mit einer anderen Flüssigkeit als Blut gefüllt, wobei jeweils die Intensität der ausgekoppelten elektromagnetischen Strahlung gemessen wird. Aus den Messwerten wird dann der Zusammenhang bestimmt, der durch eine mathematische Funktion oder durch eine Vielzahl von Wertepaaren beschrieben wird.

Das erfindungsgemäße Verfahren und die erfindungsgemäße Vorrichtung sehen vor, unterschiedlichen Schlauchleitungen verschiedene Korrekturfaktoren zuzuordnen. Wenn eine Zuordnung zwischen einem bestimmten Korrekturfaktor und einem bestimmten Schlauchleitungstyp besteht, kann von dem ermittelten Korrekturfaktor auf den verwendeten Schlauchleitungstyp geschlossen werden. Hierzu ist es lediglich erforderlich, den ermittelten Korrekturfaktor mit einem dem jeweiligen Schlauchleitungstyp zugeordneten Referenzwert zu verglichen. Wenn der ermittelte Korrekturfaktor dem jeweiligen Referenzwert entspricht oder die Abweichung von dem Referenzwert innerhalb vorgegebener Grenzen liegt, wird darauf geschlossen, dass der jeweilige Schlauchleitungstyp verwendet wird. Folglich kann mit dem erfindungsgemäßen Verfahren auch auf den verwendeten Schlauchleitungstyp geschlossen werden.

Entsprechend können auch Fremdfabrikate erkannt werden, wenn sich der ermittelte Korrekturfaktor keinem bekannten Typ einer Schlauchleitung zuordnen lässt. Es ist auch möglich, dass bei Verwendung eines Fremdfabrikats nicht nur die Anzeige der Messwerte des bestimmten Blutbestandteils deaktiviert wird, sondern auch auf einem Speichermedium gespeichert wird, dass die Behandlung mit einer unbekannten Schlauchleitung durchgeführt worden ist.

Dieses Speichermedium kann beispielsweise eine Patientenkarte oder ein interner Speicher der Dialysemaschine, beispielsweise ein FLASH-Speicher, oder ein Speichermedium eines Zentralrechners, beispielsweise eine Festplatte, sein, welcher mit der Dialysemaschine verbunden ist und die Dialysebehandlung dokumentiert.

Eine bevorzugte Ausführungsform sieht vor, den ermittelten Korrekturfaktor mit einem vorgegebenen Wert zu vergleichen. Wenn der Korrekturfaktor von dem vorgegebenen Wert um einen vorgegebenen Betrag abweichen sollte, kann daraus geschlossen werden, dass die verwendete Schlauchleitung um ein nicht mehr tolerierbares Maß von der idealen Schlauchleitung abweicht. Beispielsweise kann festgestellt werden, ob die Schlauchleitung für die Bestimmung der Konzentration des Blutbestandteils mit der erforderlichen Genauigkeit noch geeignet ist. Wenn dies nicht der Fall ist, wird vorzugsweise ein Steuersignal erzeugt, mit dem beispielsweise ein Eingriff in die Steuerung der extrakorporalen Behandlungsvorrichtung, insbesondere Hämodialysemaschine, vorgenommen oder ein Alarm ausgelöst werden kann. Beispielsweise kann angezeigt werden, dass die verwendete Schlauchleitung zur Messung des Blutbestandteils ungeeignet ist und die Anzeige des entsprechenden Messwertes des Blutbestandteils kann verhindert werden.

Es kann vorkommen, dass die verwendete Schlauchleitung durch unsachgemäße Lagerung oder durch einen Produktionsfehler ihre optischen Eigenschaften verändert, beispielsweise durch Eintrübung. Auch solche Veränderungen können erfasst werden. Es ist auch möglich, dass der Maschine der Typ der verwendeten Schlauchleitung beispielsweise durch Eingabe bekannt gemacht wird. Liegt der ermittelte Korrekturfaktor außerhalb eines Intervalls um einen für diese bekannte Schlauchleitung in der Maschine abgespeicherten Erwartungswerts für den Korrekturfaktor, kann auf einen Produktionsfehler oder eine unsachge mäße Lagerung geschlossen werden. In diesem Fall erfolgt eine entsprechende Warnung an das Bedienpersonal, beispielsweise durch eine Anzeige auf einem Display, und/oder Einriffe in die Maschinensteuerung können erfolgen.

Die elektromagnetische Strahlung, mit der die dem Fachmann bekannte Messung der Intensität vorgenommen wird, liegt vorzugsweise nicht im sichtbaren Bereich des Lichts, d.h. außerhalb einer Wellenlänge von 380 nm bis 780 nm. Besonders bevorzugt ist eine Wellenlänge von 805 nm.

Für die Bestimmung der Konzentration des Blutbestandteils ist grundsätzlich unerheblich, ob reflektierte, transmittierte und/oder gestreute elektromagnetische Strahlung gemessen wird. Entscheidend ist aber, dass die elektromagnetische Strahlung durch die Wandung der Schlauchleitung hindurchtritt.

Ein besonders bevorzugter Anwendungsfall ist die Bestimmung der Hämoglobinkonzentration. Zur Bestimmung der Hämoglobinkonzentration wird die Intensität der seitwärts gestreuten elektromagnetischen Strahlung gemessen. Ein derartiges Messverfahren ist beispielsweise in der WO 2008/000433 A1 beschrieben.

Im Folgenden wird ein Ausführungsbeispiel der Erfindung unter Bezugnahme auf die Zeichnungen im Einzelnen beschrieben.

Es zeigen:

Fig. 1 eine Vorrichtung zur extrakorporalen Blutbehandlung zusammen mit einer Vorrichtung zum Bestimmen der Konzentration eines Blutbestandteils, insbesondere die Hämoglobinkonzentration, in stark vereinfachter schematischer Darstellung,

Fig. 2 die Mittel zum Ein- und Auskoppeln von elektromagnetischer Strahlung in stark vereinfachter schematischer Darstellung,

Fig. 3 die Intensität der aus einer mit Blut gefüllten Schlauchleitung ausgekoppelten elektromagnetischen Strahlung in Abhängigkeit von der Hämoglobinkonzentration für eine Mehrzahl von unterschiedlichen Schlauchleitungen,

Fig. 4 für eine Mehrzahl von unterschiedlichen Schlauchleitungen die Abhängigkeit der Intensität einer mit Blut gefüllten und der mit einer anderen Flüssigkeit als Blut gefüllten Schlauchleitung,

Fig. 5 eine Tabelle zur Veranschaulichung der Effektivität der Korrektur bei einer bestimmten Konzentration des Blutbestandteils für unterschiedliche Schlauchleitungen und

Fig. 6 eine Tabelle zur Veranschaulichung der Effektivität der Korrektur für eine Schlauchleitung bei unterschiedlichen Konzentrationen des Blutbestandteils.

Fig. 1 zeigt nur die für die Erfindung wesentlichen Komponenten einer Vorrichtung zur extrakorporalen Blutbehandlung in stark vereinfachter schematischer Darstellung. Die extrakorporale Blutbehandlungsvorrichtung, beispielsweise Dialysevorrichtung, verfügt über einen Dialysator oder Filter 1, der durch eine semipermeable Membran 2 in eine Blutkammer 3 und eine Dialysierflüssigkeitskammer 4 unterteilt ist. Von dem Patienten führt eine arterielle Blutleitung 5 zu der Blutkammer, während von der Blutkammer eine venöse Blutleitung 6 abgeht, die zu dem Patienten führt. Eine in der arteriellen Blutleitung 5 angeordnete Blutpumpe 7 fördert das Blut im extrakorporalen Blutkreislauf I.

Das Dialysierflüssigkeitssystem II der Dialysevorrichtung ist nur andeutungsweise dargestellt. Es umfasst eine zu der Dialysierflüssigkeitskammer 4 führende Dialysierflüssigkeitszuführleitung 8 und eine von der Dialysierflüssigkeitskammer 4 abgehende Dialysierflüssigkeitsabführleitung 9.

Bei der arteriellen und venösen Blutleitung 5, 6 handelt es sich um Schlauchleitungen, die für elektromagnetische Strahlung, insbesondere für elektromagnetische Strahlung einer Wellenlänge, die nicht im sichtbaren Bereich von 380 nm bis 780 nm und besonders bevorzugt bei einer Wellenlänge von 805 nm liegt, zumindest teilweise durchlässig ist. Darüber hinaus verfügt die Blutbehandlungsvorrichtung über eine zentrale Steuereinheit 10, mit der die einzelnen Komponenten, beispielsweise die Blutpumpe 7, gesteuert werden. Die erfindungsgemäße Vorrichtung 11 zum Bestimmen der Konzentration von bestimmten Blutbestandteilen im Blut des Patienten kann Bestandteil der extrakorporalen Blutbehandlungsvorrichtung sein oder eine separate Einheit bilden. Wenn die erfindungsgemäße Vorrichtung 11 Bestandteil der Blutbehandlungsvorrichtung ist, kann sie von den Komponenten Gebrauch machen, die in der Blutbehandlungsvorrichtung ohnehin vorhanden sind.

Die Vorrichtung 11 zum Bestimmen der Konzentration eines Blutbestandteils verfügt über in Fig. 1 nur andeutungsweise dargestellte Mittel 12 zum Einkoppeln und Auskoppeln von elektromagnetischer Strahlung, die Bestandteil einer Messanordnung sind. Darüber hinaus verfügt die Vorrichtung 11 über eine Rechen- und Auswerteinheit 13, die über eine Datenleitung 14 mit der zentralen Steuereinheit 10 der Blutbehandlungsvorrichtung Daten austauschen kann.

Fig. 2 zeigt in stark vereinfachter schematischer Darstellung einen Schnitt durch die Messanordnung 15 mit den Mitteln 12 zum Einkoppeln (12A) und Auskoppeln (12B) von elektromagnetischer Strahlung. Eine derartige Messanordnung 15 für die Bestimmung der Konzentration eines Blutbestandteils, bspw. Hämoglobin, ist dem Fachmann bekannt. Die Messanordnung 15 weist eine Vorrichtung 16 zum Einspannen der Schlauchleitung 5 auf. Die Schlauchleitung 5 ist in der Vorrichtung 16 derart eingespannt, dass elektromagnetische Strahlung an planaren Grenzflächen 15A, 15B, 15C in die Schlauchleitung eintreten bzw. austreten kann. Die bekannten Messanordnungen erlauben die Messung von im Blut reflektierter, transmittierter oder gestreuter elektromagnetischer Strahlung. Die elektromagnetische Strahlung wird von einer oder mehreren Lichtemittern 12A, bspw. LED's, emittiert und von einem oder mehreren Lichtdetektoren, bspw. Fotodioden, empfangen. Es sind Messanordnungen mit vier Lichtemittern und acht Lichtdetektoren bekannt, um die optische Reflektion/Rückwärtsstreuung (BS), Transmission/Vorwärtsstreuung (FS) und/oder Seitwärtsstreuung (SS) erfassen zu können. Die Ausgangssignale der einzelnen Lichtdetektoren 12B können einzeln oder in Kombination ausgewertet werden, um den Blutbestandteil zu bestimmen. Nachfolgend wird davon ausgegangen, dass nur die Seitswärtsstreuung (SS) erfasst wird, wobei das SS-Ausgangssignal des Lichtdetektors oder der Lichtdetektoren ausgewertet wird.

Die Konzentration von Hämoglobin (HB) als Blutbestandteil kann wie folgt gemessen werden:

$$Hb = A \cdot SS^{-B} \qquad \text{(Gleichung 1)}$$

wobei A und B Konstanten sind, die bei einer werkseitigen Kalibrierung der Messanordnung festgelegt werden. Die Konstanten A und B berücksichtigen nicht den Einfluss der Schlauchleitung auf die Bestimmung des Blutparameters.

Fig. 2 zeigt, dass die elektromagnetische Strahlung durch die Schlauchleitung 5 in das Blut eingekoppelt und durch die Schlauchleitung aus dem Blut ausgekoppelt wird. Folglich tritt die elektromagnetische Strahlung zweimal durch die Wand der Schlauchleitung hindurch. Da die Schlauchleitung das Licht absorbiert und streut, wird die Messung verfälscht. Je dicker die Schlauchwand ist, desto kleiner ist das Volumen an Blut, das die elektromagnetische Strahlung absorbiert. Folglich wird die gemessene Intensität der aus der Schlauchleitung ausgekoppelten elektromagnetischen Strahlung mit zunehmender Wandstärke größer und mit abnehmender Wandstärke kleiner. Neben den geometrischen Abmessungen üben auch die Oberflächenbeschaffenheit der Schlauchleitung sowie die Materialeigenschaften oder das verwendete Sterilisationsverfahren einen Einfluss auf die Messung des Blutbestandteils aus.

Fig. 3 zeigt die Abhängigkeit der Signalamplitude des SS-Signals von der Hämoglobinkonzentration für unterschiedliche Schlauchleitungen, die sich in der Wandstärke unterscheiden. Die erste Schlauchleitung hat eine Wandstärke von 1,16 mm, die zweite Schlauchleitung 1,31 mm und die dritte Schlauchleitung 1,44 mm. Es zeigt sich, dass mit zunehmender Wandstärke die Signalamplitude des SS-Signals zunimmt. Eine Toleranz von +/- 0,1 der Schlauchwandstärke führt zu einem Hb-Messfehler von ca. +/-0,5 g/dl.

Die Rechen- und Auswerteinheit 13 der erfindungsgemäßen Vorrichtung 11 zur Bestimmung der Konzentration eines Blutbestandteils, insbesondere Hämoglobin (Hb), weist zur Minimierung oder Kompensierung des Einflusses der Schlauchleitung auf die Bestimmung der Konzentration des Blutbestandteils eine Einrichtung 13A zur Bestimmung eines Korrekturfaktors *k* auf. Die Hämoglobinkonzentration wird in der Rechen- und Auswerteinheit 13 wie folgt bestimmt:

$$Hb = k \cdot A \cdot SS^{-B} \qquad \text{(Gleichung 2)}$$

Nachfolgend wird die Berechnung des Korrekturfaktors *k* in der Auswert- und Recheneinheit 13 im Einzelnen beschrieben.

Zunächst wird für eine Mehrzahl von Schlauchleitungen die Abhängigkeit der Intensität der aus einer mit Blut gefüllten Schlauchleitung ausgekoppelten elektromagnetischen Strahlung von der Intensität der elektromagnetischen Strahlung bestimmt, die aus derselben mit einer anderen Flüssigkeit als Blut gefüllten Schlauchleitung ausgekoppelt wird. Die Intensität der ausgekoppelten elektromagnetischen Strahlung ist proportional zu dem mit der Messanordnung gemessenen SS-Signal. Die Festlegung dieses Zusammenhangs erfolgt für eine vorgegebene Konzentration des Blutbestandteils.

Fig. 4 zeigt die Signalamplitude des gemessenen SS-Signals bei einer mit Blut gefüllten Schlauchleitung als Funktion der gemessenen Signalamplitude des SS-Signals, wenn dieselbe Schlauchleitung mit einer anderen Flüssigkeit als Blut gefüllt ist. Im vorliegenden Ausführungsbeispiel ist die Flüssigkeit eine isotonische Kochsalzlösung (NaCl). Die vorgegebene Konzentration des Hämatokrit des Bluts ist bei dem vorliegenden Ausführungsbeispiel 11 g/dl.

Die einzelnen Schlauchleitungen unterscheiden sich in den geometrischen Abmessungen, insbesondere der Wandstärke und/oder den Materialeigenschaften und/oder den Sterilisationsverfahren. Zur Festlegung des Zusammenhangs zwischen den beiden Signalamplituden wird jede Schlauchleitung mit Blut und NaCl gefüllt, wobei das SS-Signal bei der mit Blut gefüllten und das SS-Signal bei der mit NaCl gefüllten Schlauchleitung gemessen wird.

Der in Fig. 4 dargestellte Zusammenhang wird durch die nachfolgende Gleichung beschrieben:

$$SS_{NaCl \rightarrow Hb11} = m \cdot SS_{NaCl} + n \qquad \text{(Gleichung 3)}$$

wobei $SS_{NaCl}$ das bei NaCl gemessene SS-Signal, $SS_{NaCl \rightarrow HB11}$ das bei Blut gemessene SS-Signal und *m, n* Konstanten sind, die Steigung und Offset der durch die Messwerte gelegten Geraden angeben. Aus den Messwerten werden die Konstanten *m* und *n* bestimmt, die in dem Speicher gespeichert werden.

Die Konstanten der Gleichung (3) sind in einem Speicher der Rechen- und Auswerteinheit 13 gespeichert, so dass die Rechen- und Auswerteinheit 13 nach Gleichung (3) aus der Signalamplitude des bei der mit NaCl gefüllten Schlauchleitung gemessenen Signals die Signalamplitude des Signals berechnen kann, die gemessen wird, wenn die Schlauchleitung mit Blut gefüllt ist, wobei die Hämatokritkonzentration Hb = 11 g/dl ist.

Die Rechen- und Auswerteinheit 13 berechnet nunmehr den Korrekturfaktor k wie folgt. Vor Beginn der Blutbehandlung wird während der Spülphase, in der die Blutleitung 5 mit einer Spülflüssigkeit, insbesondere isotonische Kochsalzlösung, oder Dialysierflüssigkeit gespült wird, mit der Messanordnung 15 die Signalamplitude $SS_{NaCl}$ gemessen. Der Messwert $SS_{NaCl}$ wird in dem Speicher der Rechen- und Auswerteinheit 13 abgelegt. Die Rechen- und Auswerteinheit 13 berechnet aus dem Messwert $SS_{NaCl}$ nach Gleichung (3) mit den gespeicherten Konstanten *m*, *n* die Signalamplitude $SS_{NaCl \rightarrow Hb11}$.

Mit Gleichung (1) wird für Hb = 11 g/dl der Wert der Signalamplitude $SS_{HB11}$ berechnet, wobei die in einer werkseitigen Kalibriermessung ermittelten Parameter A und B, die nicht den Einfluss der Schlauchleitung auf die Bestimmung der Konzentration des Blutbestandteils berücksichtigen, aus dem Speicher der Rechen- und Auswerteinheit 13 ausgelesen werden.

Nachdem die Werte für die Signalamplituden $SS_{NaCl \rightarrow Hb11}$ und $SS_{HB11}$ bekannt sind, berechnet die Rechen- und Auswerteinheit 13 den Korrekturfaktor *k* wie folgt

$$k = \frac{A \cdot {SS_{Hb11}}^{-B}}{A \cdot {SS_{NaCl \rightarrow Hb11}}^{-B}} = \left( \frac{SS_{Hb11}}{SS_{NaCl \rightarrow Hb11}} \right)^{-B} \qquad \text{(Gleichung 4)}$$

Der berechnete Korrekturfaktor *k* wird in der Rechen- und Auswerteinheit 13 mit einem vorgegebenen Wert verglichen, wobei die Rechen- und Auswerteinheit feststellt, ob der Korrekturfaktor von dem vorgegebenen Wert um einen vorgegebenen Betrag abweicht.

Im vorliegenden Ausführungsbeispiel stellt die Rechen- und Auswerteinheit 13 fest, ob der Korrekturfaktor $k = 1$, $k < 1$ oder $k > 1$ ist. Wenn der Korrekturfaktor $k = 1$ ist, entspricht die verwendete Schlauchleitung der Standardreferenz-Schlauchleitung. Wenn der Korrekturfaktor kleiner oder größer 1 ist, entspricht die Schlauchleitung nicht der Standardreferenz-Schlauchleitung. Wenn der Betrag der Differenz zwischen dem Korrekturfaktor und 1 größer als ein vorgegebener Wert, bspw. 0,2 ist, stellt die Rechen-und Auswerteinheit 13 fest, dass die Abweichungen zur Standardreferenz-Schlauchleitung groß sind. In diesem Fall erzeugt die Rechen- und Auswerteinheit 13 ein Steuersignal, das die zentrale Steuereinheit 10 der Blutbehandlungsvorrichtung über die Datenleitung 14 empfängt. Die zentrale Steuereinheit 10 kann dann einen Eingriff in die Steuerung der extrakorporalen Blutbehandlungsvorrichtung, insbesondere Hämodialysevorrichtung, vornehmen. Darüber hinaus kann ein Alarm gegeben werden. Beispielsweise kann angezeigt werden, dass die verwendete Schlauchleitung zur Messung des Blutbestandteils ungeeignet ist und die Anzeige des entsprechenden Messwertes des Blutbestandteils kann verhindert werden.

Nach dem Spülen der Blutleitungen mit einer Spülflüssigkeit oder Dialysierflüssigkeit wird die Blutbehandlung durchgeführt. Während der Blutbehandlung sind die Schlauchleitungen mit Blut gefüllt.

Die Konzentration eines Bestandteils im Blut, insbesondere des Hämoglobins, wird während der Blutbehandlung nach der folgenden Gleichung berechnet

$$Hb = \left( \frac{SS_{Hb11}}{SS_{NaCl \rightarrow Hb11}} \right)^{-B} \cdot A \cdot SS^{-B}$$

(Gleichung 5)

Nach Umformung ergibt sich

$$Hb = A \cdot (\alpha \cdot SS)^{-B}$$

(Gleichung 6)

$$\alpha = \frac{SS_{Hb11}}{SS_{NaCl \rightarrow Hb11}}$$

(Gleichung 7)

Die Konzentration des Hämoglobins wird während der Blutbehandlung von der Rechen-und Auswerteinheit 13 nach Gleichung (5) oder den Gleichungen (6) und (7) fortlaufend berechnet und auf einer Anzeigeeinheit 13B angezeigt.

Die Tabelle in Fig. 5 zeigt das Ergebnis der Korrektur. Die Tabelle zeigt die Effektivität der Korrektur bei zehn verschiedenen Schlauchleitungen, die sich in der Wandstärke, den Materialeigenschaften (Weichmacher) und den verwendeten Sterilisationsverfahren voneinander unterscheiden, wobei für die Konzentration des Hämaglobin ein fester Wert von 11 g/dl angenommen wird. Es zeigt sich, dass mit der Korrektur der Einfluss der Schlauchleitung auf die Bestimmung des Hämoglobins um mindestens 42 % verbessert wird. Für die Schlauchleitungen mit den Bezeichnungen 08A2173 und 08A3206 wird keine Korrektur vorgenommen, da der Korrekturfaktor mit $k$ = 0,9917 und $k$ = 0,9833 nur geringfügig von dem Korrekturfaktor $k$ = 1 abweicht. Wenn $1{,}05 \geq k \geq 0{,}95$ ist, wird eine Korrektur nicht durchgeführt, da eine Korrektur nicht notwendig ist.

Die Tabelle in Fig. 6 zeigt die Effektivität der Korrektur für nur eine Schlauchleitung, wobei die Konzentration des Hämoglobins zwischen 8,4 g/dl und 13,9 g/dl liegt. Es zeigt sich, dass die Effektivität der Korrektur in dem Hb-Bereich von 8,4 g/dl und 13,9 g/dl nicht konstant ist. In der Praxis wird eine Konzentration des Hämoglobins angestrebt, die zwischen 10 bis 12 g/dl liegt. In diesem Bereich liegt die Verbesserung bei durchschnittlich 64 %.

**Patentansprüche**

1. Verfahren zum Bestimmen der Konzentration eines Bestandteils von Blut in einer Schlauchleitung, die abwechselnd mit Blut und mit einer anderen Flüssigkeit gefüllt werden kann, insbesondere einer Schlauchleitung eines extrakorporalen Blutkreislaufs einer extrakorporalen Blutbehandlungsvorrichtung, wobei elektromagnetische Strahlung durch die Schlauchleitung in das Blut eingekoppelt und die durch die Schlauchleitung aus dem Blut ausgekoppelte elektromagnetische Strahlung ($SS_{Hb}$) gemessen wird, und auf der Grundlage eines die Abhängigkeit der Konzentration des Blutbestandteils von der Intensität der ausgekoppelten elektromagnetischen Strahlung beschreibenden Zusammenhangs die Konzentration des Blutbestandteils bestimmt wird,
ein Korrekturfaktor (k) für den Einfluss der Schlauchleitung auf die Bestimmung der Konzentration des Blutbestandteils ermittelt wird, und die Konzentration des Blutbestandteils auf der Grundlage des die Abhängigkeit der Konzentration des Blutbestandteils von der Intensität der ausgekoppelten elektromagnetischen Strahlung beschreibenden Zusammenhangs unter Berücksichtigung des Korrekturfaktors bestimmt wird, zur Bestimmung des Korrekturfaktors der Zusammenhang zwischen der Intensität der ausgekoppelten elektromagnetischen Strahlung ($SS_{Hb11}$) bei der Schlauchleitung, die mit Blut gefüllt ist, das den Blutbestandteil in einer bestimmten Konzentration enthält, und der Schlauchleitung, die mit einer anderen Flüssigkeit als Blut gefüllt ist ($SS_{NaCl}$), bestimmt wird, die Schlauchleitung mit der anderen Flüssigkeit als Blut gefüllt und die Intensität der ausgekoppelten elektromagnetischen Strahlung gemessen wird, und der Korrekturfaktor auf der Grundlage des ermittelten Zusammenhangs zwischen der Intensität der ausgekoppelten elektromagnetischen Strahlung bei der Schlauchleitung, die mit Blut gefüllt ist, und der Schlauchleitung, die mit einer anderen Flüssigkeit als Blut gefüllt ist, und auf der Grundlage der gemessenen Intensität der elektromagnetischen Strahlung, die aus der mit einer anderen Flüssigkeit als Blut gefüllten Schlauchleitung ausgekoppelt wird, ermittelt wird,
unterschiedlichen Typen von Schlauchleitungen Referenzwerte für den Korrekturfaktor zugeordnet werden,
und der ermittelte Korrekturfaktor mit den Referenzwerten, die den unterschiedlichen Typen von Schlauchleitungen zugeordnet sind, verglichen wird, wobei auf den verwendeten Schlauchleitungstyp geschlossen wird, wenn der ermittelte Korrekturfaktor dem jeweiligen Referenzwert entspricht oder die Abweichung von dem Referenzwert innerhalb vorgegebener Grenzen liegt.

2. Verfahren nach Anspruch 1, **dadurch gekennzeichnet, dass** der Zusammenhang zwischen der Intensität der ausgekoppelten elektromagnetischen Strahlung bei der Schlauchleitung, die mit Blut gefüllt ist, das den Blutbestandteil in einer bestimmten Konzentration enthält, und der Schlauchleitung, die mit einer anderen Flüssigkeit als Blut gefüllt ist, durch eine mathematische Funktion beschrieben wird.

3. Verfahren nach Anspruch 1 oder 2, **dadurch gekennzeichnet, dass** zur Bestimmung des Zusammenhangs zwischen der Intensität der ausgekoppelten elektromagnetischen Strahlung bei der Schlauchleitung, die mit Blut gefüllt ist, das den Blutbestandteil in einer bestimmten Konzentration enthält, und der Schlauchleitung, die mit einer anderen Flüssigkeit als Blut gefüllt ist, eine Mehrzahl von unterschiedlichen Schlauchleitungen mit Blut gefüllt werden, wobei jeweils die Intensität der ausgekoppelten elektromagnetischen Strahlung gemessen wird, und die Schlauchleitungen mit der anderen Flüssigkeit gefüllt werden, wobei jeweils die Intensität der ausgekoppelten elektromagnetischen Strahlung gemessen wird, und der Zusammenhang aus den Messwerten bestimmt wird.

4. Verfahren nach einem der Ansprüche 1 bis 3, **dadurch gekennzeichnet, dass** der Korrekturfaktor mit einem vorgegebenen Wert verglichen wird, wobei ein Steuersignal erzeugt wird, wenn der Korrekturfaktor von dem vorgegebenen Wert um einen vorgegebenen Betrag abweicht.

5. Verfahren nach einem der Ansprüche 1 bis 4, **dadurch gekennzeichnet, dass** die andere Flüssigkeit als Blut, mit der die Schlauchleitung gefüllt wird, eine Dialysierflüssigkeit oder eine Spülflüssigkeit, insbesondere eine isotonische Kochsalzlösung, ist.

6. Verfahren nach einem der Ansprüche 1 bis 5, **dadurch gekennzeichnet, dass** die elektromagnetische Strahlung eine Wellenlänge hat, die bevorzugt nicht im sichtbaren Bereich von 380 nm bis 780 nm liegt und besonders bevorzugt bei 805 nm liegt.

7. Verfahren nach einem der Ansprüche 1 bis 6, **dadurch gekennzeichnet, dass** der Blutbestandteil Hämoglobin ist

8. Vorrichtung zur Bestimmung der Konzentration eines Bestandteils von Blut in einer Schlauchleitung, die abwechselnd mit Blut und mit einer anderen Flüssigkeit gefüllt werden kann

   insbesondere einer Schlauchleitung eines extrakorporalen Blutkreislaufs einer extrakorporalen Blutbehandlungsvorrichtung, mit Mitteln (12A) zum Einkoppeln von elektromagnetischer Strahlung durch die Schlauchleitung in das Blut,
   Mitteln (12B) zum Messen der durch die Schlauchleitung aus dem Blut ausgekoppelten elektromagnetische Strahlung,
   einer Rechen- und Auswerteinheit (13), die derart ausgebildet ist, dass auf der Grundlage eines die Abhängigkeit der Konzentration des Blutbestandteils von der Intensität der ausgekoppelten elektromagnetischen Strahlung beschreibenden Zusammenhangs die Konzentration des Blutbestandteils bestimmt wird, wobei
die Rechen- und Auswerteinheit (13) eine Einrichtung (13A) zur Bestimmung eines Korrekturfaktors für den Einfluss der Schlauchleitung auf die Bestimmung der Konzentration des Blutbestandteils aufweist,
die Einrichtung (13A) zur Bestimmung eines Korrekturfaktors Mittel zum Festlegen des Zusammenhangs zwischen der Intensität der ausgekoppelten elektromagnetischen Strahlung bei der Schlauchleitung, die mit Blut gefüllt ist, das den Blutbestandteil in einer bestimmten Konzentration enthält, und der Schlauchleitung, die mit einer anderen Flüssigkeit als Blut gefüllt ist, aufweist, und die Einrichtung (13A) zur Bestimmung des Korrekturfaktors derart ausgebildet ist,
dass der Korrekturfaktor auf der Grundlage des ermittelten Zusammenhangs zwischen der Intensität der ausgekoppelten elektromagnetischen Strahlung bei einer der Schlauchleitung, die mit Blut gefüllt ist, und der Schlauchleitung, die mit einer anderen Flüssigkeit als Blut gefüllt ist, und auf der Grundlage der gemessenen Intensität der elektromagnetischen Strahlung, die aus der mit einer anderen Flüssigkeit als Blut gefüllten Schlauchleitung ausgekoppelt wird, bestimmt wird,
die Einrichtung (13A) zur Bestimmung des Korrekturfaktors derart ausgebildet ist, dass unterschiedlichen Typen von Schlauchleitungen Referenzwerte für den Korrekturfaktor zugeordnet sind,
und der ermittelte Korrekturfaktor mit den Referenzwerten, die den unterschiedlichen Typen von Schlauchleitungen zugeordnet sind, verglichen wird, wobei
auf den verwendeten Schlauchleitungstyp geschlossen wird, wenn der ermittelte Korrekturfaktors dem jeweiligen Referenzwert entspricht oder die Abweichung von dem Referenzwert innerhalb vorgegebener Grenzen liegt.

**9.** Vorrichtung nach Anspruch 8, **dadurch gekennzeichnet, dass** die Rechen- und Auswerteinheit (13) derart ausgebildet ist, dass der Zusammenhang zwischen der Intensität der ausgekoppelten elektromagnetischen Strahlung bei einer der Schlauchleitung, die mit Blut gefüllt ist, das den Blutbestandteil in einer bestimmten Konzentration enthält, und der Schlauchleitung, die mit einer anderen Flüssigkeit als Blut gefüllt ist, durch eine mathematische Funktion beschrieben wird.

**10.** Vorrichtung nach Anspruch 8 oder 9, **dadurch gekennzeichnet, dass** die Rechen-und Auswerteinheit (13) derart ausgebildet ist, dass der Korrekturfaktor mit einem vorgegebenen Wert verglichen wird, wobei ein Steuersignal erzeugt wird, wenn der Korrekturfaktor von dem vorgegebenen Wert um einen vorgegebenen Betrag abweicht.

**11.** Vorrichtung nach einem der Ansprüche 8 bis 10, **dadurch gekennzeichnet, dass** die andere Flüssigkeit als Blut, mit der die Schlauchleitung gefüllt wird, eine Dialysierflüssigkeit oder eine Spülflüssigkeit, insbesondere eine isotonische Kochsalzlösung, ist.

**12.** Vorrichtung nach einem der Ansprüche 8 bis 11, **dadurch gekennzeichnet, dass** die elektromagnetische Strahlung eine Wellenlänge hat, die bevorzugt nicht im sichtbaren Bereich von 380 nm bis 780 nm liegt und besonders bevorzugt bei 805 nm liegt.

**13.** Vorrichtung nach einem der Ansprüche 8 bis 12, **dadurch gekennzeichnet, dass** der Blutbestandteil Hämoglobin ist.

**14.** Vorrichtung zur extrakorporalen Blutbehandlung mit einem extrakorporalen Blutkreislauf (I) und einem Dialysierflüssigkeitssystem (II), wobei der extrakorporale Blutkreislauf eine venöse und eine arterielle Schlauchleitung (5, 6) aufweist, **dadurch gekennzeichnet, dass** die extrakorporale Blutbehandlungsvorrichtung eine Vorrichtung zur Bestimmung der Konzentration eines Bestandteils von Blut nach einem der Ansprüche 8 bis 13 aufweist.

## Claims

**1.** A method for determining the concentration of a constituent of blood in a hose line, which can be alternately filled with blood and another fluid, in particular a hose line of an extracorporeal blood circuit of an extracorporeal blood treatment apparatus, wherein
electromagnetic radiation is coupled by the hose line into the blood and the electromagnetic radiation ($SS_{Hb}$) decoupled by the hose line from the blood is measured, and the concentration of the blood constituent is determined on the basis of a relationship describing the dependence of the concentration of the blood constituent on the intensity of the decoupled electromagnetic radiation,
a correction factor (k) is ascertained for the influence of the hose line on the determination of the concentration of the blood constituent, and the concentration of the blood constituent is determined on the basis of the relationship describing the dependence of the concentration of the blood constituent on the intensity of the decoupled electromagnetic radiation, account being taken of the correction factor,
in order to determine the correction factor, the relationship between the intensity of the decoupled electromagnetic radiation ($SS_{Hb11}$) in the hose line filled with blood containing the blood constituent in a specific concentration and the hose line filled with a fluid other than blood ($SS_{NaCL}$) is determined,
the hose line is filled with a fluid other than blood and the intensity of the decoupled electromagnetic radiation is measured, and the correction factor is determined on the basis of the ascertained relationship between the intensity of the decoupled electromagnetic radiation in the hose line filled with blood and the hose line filled with a fluid other than blood, and on the basis of the measured intensity of the electromagnetic radiation which is decoupled from the hose line filled with a fluid other than blood,
reference values for the correction factor are assigned to different types of hose lines, and the ascertained correction factor is compared with the reference values, which are assigned to the different types of hose lines, wherein it is concluded on the type of the employed hose line, if the ascertained correction factor corresponds to the respective reference value or the deviation from the reference value lies within predetermined limits.

2. The method according to claim 1, **characterised in that** the relationship between the intensity of the decoupled electromagnetic radiation in a hose line filled with blood containing the blood constituent in a specific concentration and the hose line filled with a fluid other than blood is described by a mathematical function.

3. The method according to claim 1 or 2, **characterised in that**, in order to determine the relationship between the intensity of the decoupled electromagnetic radiation in a hose line filled with blood containing the blood constituent in a specific concentration and the hose line filled with a fluid other than blood, a plurality of different hose lines are filled with blood, the intensity of the decoupled electromagnetic radiation being measured in each case, and the hose lines are filled with the other fluid, the intensity of the decoupled electromagnetic radiation being measured in each case, and the relationship is determined from the measured values.

4. The method according to any one of claims 1 to 3, **characterised in that** the correction factor is compared with a preset value, a control signal being generated when the correction factor diverges from the preset value by a preset amount.

5. The method according to any one of claims 1 to 4, **characterised in that** the fluid other than blood, with which the hose line is filled, is a dialysing fluid or a rinsing fluid, in particular an isotonic salt solution.

6. The method according to any one of claims 1 to 5, **characterised in that** the electromagnetic radiation has a wavelength which preferably does not lie in the visible region from 380 nm to 780 nm and particularly preferably lies at 805 nm.

7. The method according to any one of claims 1 to 6, **characterised in that** the blood constituent is haemoglobin.

8. A device for determining the concentration of a constituent of blood in a hose line, which can be alternately filled with blood and another fluid, in particular a hose line of an extracorporeal blood circuit of an extracorporeal blood treatment apparatus, comprising
means (12A) for the coupling of electromagnetic radiation by the hose line into the blood,
means (12B) for measuring the electromagnetic radiation decoupled by the hose line from the blood,
a computing and evaluation unit (13), which is designed such that the concentration of the blood constituent is determined on the basis of a relationship describing the dependence of the concentration of the blood constituent on the intensity of the decoupled electromagnetic radiation, wherein
the computing and evaluation unit (13) comprises a device (13A) for determining a correction factor for the influence of the hose line on the determination of the concentration of the blood constituent,
the device (13A) for determining a correction factor comprising means for establishing the relationship between the intensity of the decoupled electromagnetic radiation in the hose line filled with blood containing the blood constituent of a specific concentration and the hose line filled with a fluid other than blood, and
the device (13A) for the determination of the correction factor being designed such that the correction factor is determined on the basis of the ascertained relationship between the intensity of the decoupled electromagnet radiation in the hose line filled with blood and the hose line filled with a fluid other than blood, and on the basis of the measured intensity of the electromagnetic radiation which is decoupled from the hose line filled with a fluid other than blood,
the device (13A) for the determination of the correction factor being designed such that reference values for the correction factor are assigned to different types of hose lines, and the ascertained correction factor is compared with the reference values, which are assigned to the different types of hose lines, wherein it is concluded on the type of the employed hose line, if the ascertained correction factor corresponds to the respective reference value or the deviation from the reference value lies within predetermined limits.

**9.** The device according to claim 8, **characterised in that** the computing and evaluation unit (13) is designed such that the relationship between the intensity of the decoupled electromagnetic radiation in the hose line filled with blood containing the blood constituent in a specific concentration and the hose line filled with a fluid other than blood is described by a mathematical function.

**10.** The device according to claim 8 or 9, **characterised in that** the computing and evaluation unit (13) is designed such that the correction factor is compared with a preset value, a control signal being generated when the correction factor diverges from the preset value by a preset amount.

**11.** The device according to any one of claims 8 to 10, **characterised in that** the fluid other than blood, with which the hose line is filled, is a dialysing fluid or a rinsing fluid, in particular an isotonic salt solution.

**12.** The device according to any one of claims 8 to 11, **characterised in that** the electromagnetic radiation has a wavelength which preferably does not lie in the visible region from 380 nm to 780 nm and particularly preferably lies at 805 nm.

**13.** The device according to any one of claims 8 to 12, **characterised in that** the blood constituent is haemoglobin.

**14.** An apparatus for extracorporeal blood treatment with an extracorporeal blood circuit (I) and a dialysing fluid system (II), wherein the extracorporeal blood circuit comprises a venous and an arterial hose line (5, 6), **characterised in that** the extracorporeal blood treatment apparatus comprises a device for determining the concentration of a constituent of blood according to any one of claims 8 to 13.

## Revendications

**1.** Procédé de détermination de la concentration d'un constituant du sang dans une conduite flexible, qui peut être remplie tour à tour de sang et d'un autre fluide,
en particulier une conduite flexible d'un circuit de sang extracorporel d'un dispositif de traitement de sang extracorporel, dans lequel un rayonnement électromagnétique est injecté dans le sang par la conduite flexible et le rayonnement électromagnétique ($SS_{Hb}$) extrait du sang par la conduite flexible est mesuré, et sur la base d'une relation décrivant la dépendance de la concentration du constituant du sang de l'intensité du rayonnement électromagnétique extrait, la concentration du constituant du sang est déterminée,
un facteur de correction (k) pour l'influence de la conduite flexible sur la détermination de la concentration du constituant du sang est déterminé, et la concentration du constituant du sang est déterminée sur la base de la relation décrivant la dépendance de la concentration du constituant du sang de l'intensité du rayonnement électromagnétique extrait en tenant compte du facteur de correction,
pour la détermination du facteur de correction, la relation entre l'intensité du rayonnement électromagnétique ($SS_{Hb11}$) extrait pour la conduite flexible, qui est remplie de sang, qui contient le constituant du sang dans une concentration déterminée, et la conduite flexible, qui est remplie d'un autre fluide que du sang ($SS_{NaCl}$), est déterminée,
la conduite flexible est remplie de l'autre fluide que du sang et l'intensité du rayonnement électromagnétique extrait est mesurée, et
le facteur de correction est déterminé sur la base de la relation déterminée entre l'intensité du rayonnement électromagnétique extrait pour la conduite flexible, qui est remplie de sang, et la conduite flexible, qui est remplie d'un autre fluide que du sang, et sur la base de l'intensité mesurée du rayonnement électromagnétique, qui est extrait de la conduite flexible remplie d'un autre fluide que du sang,
des valeurs de référence pour le facteur de correction sont affectées à différents types de conduites flexibles,
et le facteur de correction déterminé est comparé avec les valeurs de référence, qui sont affectées aux différents types de conduites flexibles,
dans lequel le type de conduite flexible utilisé est déduit, lorsque le facteur de correction déterminé correspond à la valeur de référence respective ou lorsque l'écart de la valeur de référence se trouve dans des limites prédéfinies.

**2.** Procédé selon la revendication 1, **caractérisé en ce que** la relation entre l'intensité du rayonnement électromagnétique extrait pour la conduite flexible, qui est remplie de sang, qui contient le constituant du sang dans une concentration déterminée, et la conduite flexible, qui est remplie d'un autre liquide que du sang, est décrite par une fonction mathématique.

**3.** Procédé selon la revendication 1 ou 2, **caractérisé en ce que** pour la détermination de la relation entre l'intensité du rayonnement électromagnétique extrait pour la conduite flexible, qui est remplie de sang, qui contient le constituant du sang dans une concentration déterminée, et la conduite flexible, qui est remplie d'un autre liquide que du sang, une pluralité de différentes conduites flexibles sont remplies de sang, dans lequel respectivement l'intensité du rayonnement électromagnétique extrait est mesurée, et les conduites flexibles sont remplies de l'autre fluide, dans lequel respectivement l'intensité du rayonnement électromagnétique extrait est mesurée, et la relation est déterminée à partir des valeurs mesurées.

**4.** Procédé selon l'une quelconque des revendications 1 à 3, **caractérisé en ce que** le facteur de correction est comparé avec une valeur prédéfinie, dans lequel un signal de commande est généré, lorsque le facteur de correction diverge de la valeur prédéfinie d'une valeur prédéfinie.

**5.** Procédé selon l'une quelconque des revendications 1 à 4, **caractérisé en ce que** l'autre fluide que du sang, dont la conduite flexible est remplie, est un fluide de dialyse ou un fluide de rinçage, en particulier une solution physiologique.

**6.** Procédé selon l'une quelconque des revendications 1 à 5, **caractérisé en ce que** le rayonnement électromagnétique a une longueur d'onde, qui ne se trouve de préférence pas dans la plage visible de 380 nm à 780 nm et est de manière particulièrement préférée de 805 nm.

**7.** Procédé selon l'une quelconque des revendications 1 à 6, **caractérisé en ce que** le constituant du sang est de l'hémoglobine.

**8.** Dispositif de détermination de la concentration d'un constituant du sang dans une conduite flexible, qui peut être remplie tour à tour de sang et d'un autre fluide,
en particulier une conduite flexible d'un circuit de sang extracorporel d'un dispositif de traitement de sang extracorporel, avec
des moyens (12A) d'injection de rayonnement électromagnétique dans le sang par la conduite flexible,
des moyens (12B) de mesure du rayonnement électromagnétique extrait du sang par la conduite flexible,
une unité de calcul et d'évaluation (13), qui est conçue de sorte que sur la base d'une relation décrivant la dépendance de la concentration du constituant du sang de l'intensité du rayonnement électromagnétique extrait, la concentration du constituant du sang est déterminée,
dans lequel
l'unité de calcul et d'évaluation (13) présente un dispositif (13A) de détermination d'un facteur de correction pour l'influence de la conduite flexible sur la détermination de la concentration du constituant du sang,
le dispositif (13A) de détermination d'un facteur de correction présente des moyens de détermination de la relation entre l'intensité du rayonnement électromagnétique extrait pour la conduite flexible, qui est remplie de sang, qui contient le constituant du sang dans une concentration déterminée, et la conduite flexible, qui est remplie d'un autre fluide que du sang, et
le dispositif (13A) de détermination du facteur de correction est réalisé de sorte que le facteur de correction est déterminé sur la base de la relation déterminée entre l'intensité du rayonnement électromagnétique extrait pour une parmi la conduite flexible, qui est remplie de sang, et la conduite flexible, qui est remplie d'un autre fluide que du sang, et sur la base de l'intensité mesurée du rayonnement électromagnétique, qui est extrait de la conduite flexible remplie d'un autre fluide que du sang,
le dispositif (13A) de détermination du facteur de correction est réalisé de sorte que des valeurs de référence pour le facteur de correction sont affectées à différents types de conduites flexibles,
et le facteur de correction déterminé est comparé avec les valeurs de référence, qui sont affectées aux différents types de conduites flexibles,
dans lequel
le type de conduite flexible utilisé est déduit, lorsque le facteur de correction déterminé correspond à la valeur de référence respective ou lorsque l'écart de la valeur de référence se trouve dans des limites prédéfinies.

**9.** Dispositif selon la revendication 8, **caractérisé en ce que** l'unité de calcul et d'évaluation (13) est réalisée de sorte que la relation entre l'intensité du rayonnement électromagnétique extrait pour la conduite flexible, qui est remplie de sang, qui contient le constituant du sang dans une concentration déterminée, et la conduite flexible, qui est remplie d'un autre liquide que du sang, est décrite par une fonction mathématique.

**10.** Dispositif selon la revendication 8 ou 9, **caractérisé en ce que** l'unité de calcul et d'évaluation (13) est réalisée de sorte que le facteur de correction est comparé avec une valeur prédéfinie, dans lequel un signal de commande est généré, lorsque le facteur de correction diverge de la valeur prédéfinie d'une valeur prédéfinie.

**11.** Dispositif selon l'une quelconque des revendications 8 à 10, **caractérisé en ce que** l'autre fluide que du sang, dont la conduite flexible est remplie, est un fluide de dialyse ou un fluide de rinçage, en particulier une solution physiologique.

**12.** Dispositif selon l'une quelconque des revendications 8 à 11, **caractérisé en ce que** le rayonnement électromagnétique a une longueur d'onde, qui ne se trouve de préférence pas dans la plage visible de 380 nm à 780 nm et est de manière particulièrement préférée de 805 nm.

**13.** Dispositif selon l'une quelconque des revendications 8 à 12, **caractérisé en ce que caractérisé en ce que** le constituant du sang est de l'hémoglobine.

**14.** Dispositif de traitement de sang extracorporel avec un circuit de sang extracorporel (I) et un système de fluide de dialyse (II), dans lequel le circuit de sang extracorporel présente une conduite flexible veineuse et une conduite flexible artérielle (5, 6), **caractérisé en ce que** le dispositif de traitement de sang extracorporel présente un dispositif de détermination de la concentration d'un constituant du sang selon l'une quelconque des revendications 8 à 13.

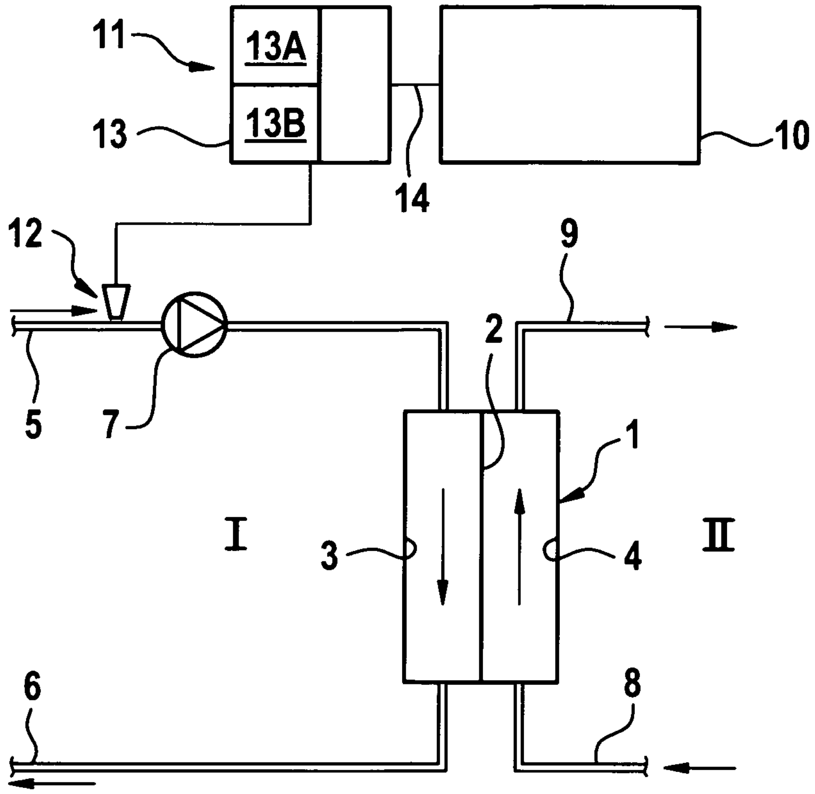
11
13A
13B
13
14
10
12
9
2
5
7
1
I
3
4
II
6
8

Fig. 1

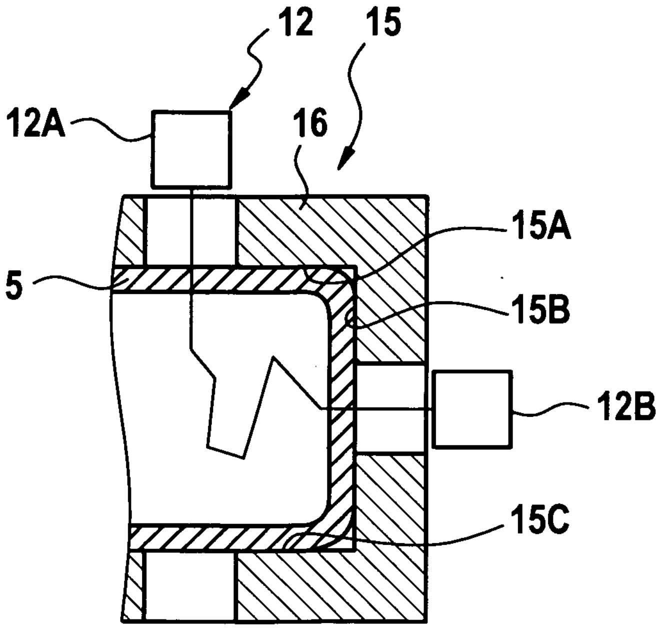
12
15
12A
16
15A
5
15B
12B
15C

Fig. 2

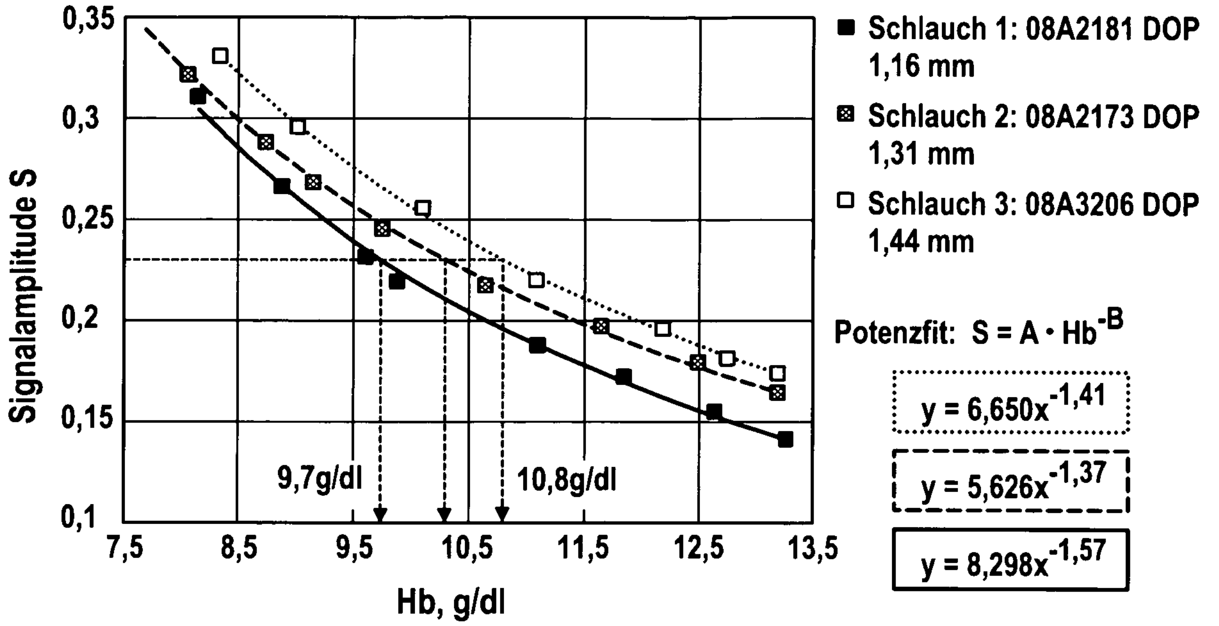
0,35
0,3
0,25
0,2
0,15
0,1
Signalamplitude S
7,5
8,5
9,5
10,5
11,5
12,5
13,5
Hb, g/dl
9,7g/dl
10,8g/dl
Schlauch 1: 08A2181 DOP 1,16 mm
Schlauch 2: 08A2173 DOP 1,31 mm
Schlauch 3: 08A3206 DOP 1,44 mm
Potenzfit: S = A • Hb^-B
y = 6,650x^-1,41
y = 5,626x^-1,37
y = 8,298x^-1,57

Fig. 3

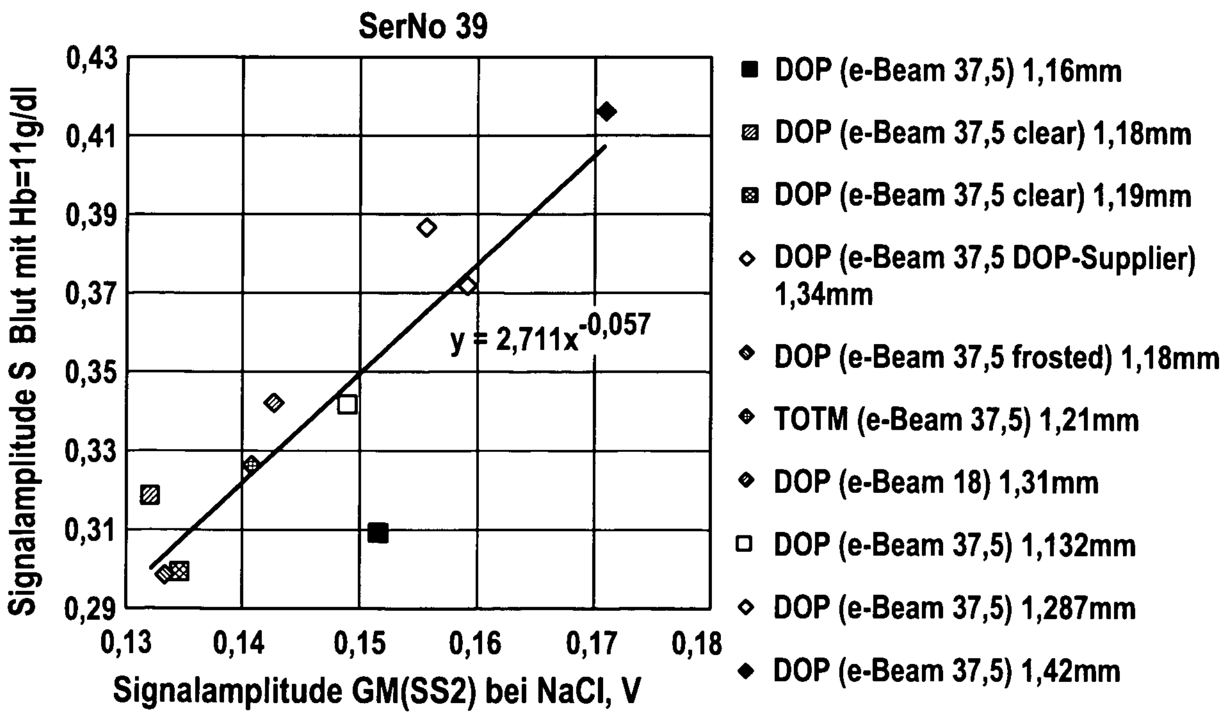
SerNo 39
0,43
0,41
0,39
0,37
0,35
0,33
0,31
0,29
Signalamplitude S Blut mit Hb=11g/dl
0,13
0,14
0,15
0,16
0,17
0,18
Signalamplitude GM(SS2) bei NaCl, V
y = 2,711x^-0,057
DOP (e-Beam 37,5) 1,16mm
DOP (e-Beam 37,5 clear) 1,18mm
DOP (e-Beam 37,5 clear) 1,19mm
DOP (e-Beam 37,5 DOP-Supplier) 1,34mm
DOP (e-Beam 37,5 frosted) 1,18mm
TOTM (e-Beam 37,5) 1,21mm
DOP (e-Beam 18) 1,31mm
DOP (e-Beam 37,5) 1,132mm
DOP (e-Beam 37,5) 1,287mm
DOP (e-Beam 37,5) 1,42mm

Fig. 4

| Schlauch-bez. | Wand-stärke, mm | Weich-macher | Sterilisation, e-Beam | Hb-Fehler | Korrek-turfak-tor, k | Hb-Fehler, mit k | Verbes-serung (grün) | Verbes-serung in % |
|---|---|---|---|---|---|---|---|---|
| 08A2181 | 1,166 | DOP | 37,5kGy | 1,4526 | 0,8281 | 0,6880 | 0,7647 | 53 |
| 08A2173 | 1,29 | DOP | 37,5kGy | 0,0028 | 0,9917 | 0,0938 | 0,0910 | |
| 08A3206 | 1,423 | DOP | 37,5kGy | 0,1240 | 0,9833 | 0,3052 | 0,1812 | |
| 08A2181 | 1,16 | DOP | 37,5kGy | 2,2093 | 0,9004 | 0,8941 | 1,3153 | 60 |
| 08A3205 | 1,18 | DOP | 37,5kGy, clear | 1,9012 | 0,8453 | 0,0941 | 1,8071 | 95 |
| 08A3275 | 1,19 | DOP | 37,5kGy, clear | 2,6905 | 0,8121 | 0,1184 | 2,5720 | 96 |
| 08A3093 | 1,34 | DOP | 37,5kGy, DOP- | 0,2102 | 0,9703 | 0,1226 | 0,0877 | 42 |
| 08A3274 | 1,18 | DOP | 37,5kGy, frosted | 2,4706 | 0,8529 | 0,4887 | 1,9819 | 80 |
| 07A3062 | 1,21 | TOTM | 37,5kGy | 1,1151 | 0,8674 | 0,4913 | 0,6237 | 56 |
| 08A2173 | 1,31 | DOP | 18kGy | 1,4424 | 0,9230 | 0,4841 | 0,9583 | 66 |

**Fig. 5**

| | Hb-Richtig, g/dl | Hb-Fehler, g/dl | | Verbesserung in % |
|---|---|---|---|---|
| | | Vor der Korrektur | Nach der Korrektur | |
| Schlauch 1 | 13,87 | 1,83 | 0,87 | 53 |
| | 13,00 | 1,61 | 0,90 | 44 |
| | 12,40 | 1,59 | 0,81 | 49 |
| | 12,00 | 1,61 | 0,73 | 55 |
| | 11,37 | 1,84 | 0,43 | 77 |
| | 10,93 | 1,53 | 0,61 | 60 |
| | 9,94 | 1,47 | 0,49 | 67 |
| | 9,63 | 1,30 | 0,57 | 56 |
| | 9,33 | 1,22 | 0,59 | 52 |
| | 8,97 | 0,97 | 0,74 | 24 |
| | 8,35 | 0,85 | 0,73 | 14 |

**Fig. 6**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2008000433 A1
- US 6718190 B2
- DE 69332325 T2
- DE 102009005402 A1